# EUROPÄISCHE PATENTSCHRIFT

(11) **EP 1 013 257 B1**
(45) Veröffentlichungstag und Bekanntmachung des Hinweises auf die Patenterteilung: **31.08.2005**
(21) Anmeldenummer: 99122831.3
(22) Anmeldetag: 17.11.1999
(51) Int. Cl.: A61K 7/06

(54) **Haarbehandlungsmittel enthaltend Oligoester**
Hair treatment compositions containing oligoesters
Composition pour le traitement des cheveux contenant des oligoesters

(30) Priorität: 25.11.1998 DE 19854352
(43) Veröffentlichungstag der Anmeldung: 28.06.2000
(73) Patentinhaber: Clariant GmbH, 65929 Frankfurt am Main (DE)
(72) Erfinder: Schuler, Wilfried, Dr., 65551 Limburg (DE)

(56) Entgegenhaltungen:
- EP-A- 0 673 638
- FR-A- 2 760 643
- FR-A- 2 781 233
- US-A- 4 785 060
- CHEMICAL ABSTRACTS, vol. 130, no. 1, 4. Januar 1999 (1999-01-04) Columbus, Ohio, US; abstract no. 5129, "Optimised soil release polymers. Basic effects, detergency benefits, and interactions with detergent ingredients" XP002169316 & LANG,FRANK-PETER: CHIM. OGGI, Bd. 16, Nr. 9, 1998, Seiten 14-18,

## Beschreibung

Die Erfindung betrifft Haarbehandlungsmittel, beispielsweise Haarschampoos, Haarspülungen, Haarkuren, mit einem Gehalt an Polyestern.

Durch oftmaliges Bleichen, Dauerwellen und Färben, aber auch durch häufiges Waschen der Haare mit entfettenden Tensiden kommt es zu einer Schädigung der Haarstruktur. Das Haar wird spröde und verliert seinen Glanz. Weiterhin lädt sich das Haar beim Kämmen elektrostatisch auf, und die angerauhte Haaroberfläche verursacht Verfilzungen sowie Verknotungen der Haare. Hierdurch wird das Kämmen erschwert. Haarbehandlungsmittel mit einer kämmbarkeitsverbessernden und pflegenden Wirkung haben daher eine erhebliche Bedeutung erlangt.

Derartige Mittel werden beispielsweise häufig in Form einer klaren Haarpflegespülung, eines Aerosolschaumes oder auch in Emulsionsform als sogenannte Creme-Rinses nach der Haarwäsche im noch nassen Haar verteilt und je nach Art des Haarbehandlungsmittels, entweder nach einigen Minuten Einwirkzeit mit Wasser ausgespült oder aber auf dem Haar belassen.
Als Wirkstoffe zur Verbesserung der Haarstruktur werden hauptsächlich kationische, insbesondere quarternäre Ammoniumverbindungen, wie Cetyltrimethylammoniumchlorid alleine oder in Kombination mit verschiedenen wachsartigen Zusätzen, wie zum Beispiel Kohlenwasserstoffen, Fettalkoholen und Fettsäuren, eingesetzt. Haarreinigung und -pflege in zwei Schritten ist zeitraubend, so daß von vielen Verbrauchern Haarmittel mit reinigender und pflegender Wirkung zugleich, bevorzugt werden.

Zur Herstellung konditionierender Schampoos stehen einer Reihe von pflegenden Wirkstoffen zur Verfügung.
Dazu zählen Öle und ölähnliche Substanzen wie beispielsweise flüssige Kohlenwasserstoffverbindungen, Fettalkohole, Monocarboxysäureester (monocarboxylic acid ester), Polyalkoholester, Silikone, sowohl lösliche, wie beispielsweise dimethicone copolyols, und unlösliche Silikone, beispielsweise Polydimethylsiloxane, sowie kationische oberfächenaktive Mittel und kationische Polymere.

In der DE-A-195 00 841 werden Haarshampoos auf Basis anionischer Tenside offenbart, die neben kationischen Polymeren und Polysiloxanen auch Alkyloligoglucoside und Proteinfettsäurekondensate aufweisen. In DE-A-44 38 115 werden Haarbehandlungsmittel vorgeschlagen, enthaltend Alkyl- und/oder Alkenyloligoglykoside und/oder Fettsäure-N-alkylpolyhydroxyalkylamide und Proteinabietinsäurekondensate. Konditionierende Schampoos, enthaltend Silikone werden in verschiedenen Patentschriften (U.S. 2 826 551, U.S. 3 964 500, U.S. 4 364 837, GB 849 433, U.S. 4 741 855, U.S. 4 788 006 und 4 902 499, No. 4 704 272 und in EP 0 461 593), beschrieben. DE 30 48 075 beschreibt die Anwendung von quarternären Polymerisaten als Pflegemittel in Haarbehandlungsmitteln.

Haarbehandlungsmittel, enthaltend oben genannte Bestandteile weisen oft den Nachteil auf, dass ihr Schaumvermögen nicht immer zufriedenstellend ist. Dies bezieht sich einerseits auf die Höhe des Basisschaums, andererseits auch auf die Schaumbeständigkeit, insbesondere in hartem Wasser. Ein weiterer Nachteil besteht darin, dass Haarschampoos nach dem Abspülen oftmals ein stumpfes Gefühl auf den Haaren hinterlassen und die Kämmbarkeit verschlechtern. Häufig eingesetzte kationische Mittel zur Haarpflege verleihen den feuchten Haaren einen klebrigen Griff und beschweren die trockenen Haare.

EP 673 638 A2 offenbart ein Haarbehandlungsmittel zur Verbesserung unterschiedlichen Haareigenschaften enthaltend ein Antikörper auf der Basis von Eigelb-Eiweiß als Wirkstoff in Kombination mit einer Emulsion eines Polymers wie ein Polyterephthat-Polyethylenglykol Polyester das selbst kein Einfluss auf Eigenschafte wie die Weichheit des Haares aufweist.

Überraschenderweise wurde gefunden, dass die Einarbeitung von Polyestern in Haarbehandlungsmittel das Schaumvermögen, die Schaumstruktur und die Trockenkämmbarkeit signifikant verbessert. Zusätzlich ist eine Reduzierung der Rückfettung der Haare, sowie eine antistatische und griffverbessernde Wirkung und ein angenehmes Hautgefühl festzustellen. Sie bewirken, daß trockene Haare locker, glänzend und leicht zu entwirren sind.

Polyester unter der Bezeichnung Soil Release Polymere (SRP) werden seit langem wegen ihrer schmutzablösenden Wirkung und zur Verbesserung der Antistatik- und

Gleiteigenschaften von Textilfasern in Wasch- und oberfächenaktiven Reinigungsmitteln sowie in der Faserpräparation, eingesetzt.

Die deutschen Offenlegungsschriften DE-A-16 17 141 und DE-A-22 00 911 beschreiben die Einarbeitung von Polyterephthalat-Polyoxyethylenglykol-Copolymeren bzw eines Mischpolymers aus Polyethylenglykol und Polyethylenterephthalates in Waschmitteln zur besseren Auswaschbarkeit von ölig/fettigen Anschmutzungen aus Baumwoll/Polyester Mischgeweben. In U.S. 3 557 039 wird die Synthese eines Polyesters ausgehend von Dimethylterephthalat und Ethylenglykol in Gegenwart eines Katalysators beschrieben, in der U.S. 3 959 280 genannten Synthese wird zusätzlich Polyethylenoxid als Reaktant verwendet. EP-A-066 944 betrifft Textilbehandlungsmittel, die einen Copolyester aus Ethylenglykol, Polyethylenglykol, aromatischer Dicarbonsäure und sulfonierter aromatischer Dicarbonsäure in bestimmten Molverhältnissen enthalten.

Gegenstand der Erfindung sind Haarbehandlungsmittel, enthaltend 0,5 bis 5 Gew-% Oligoester der Formel 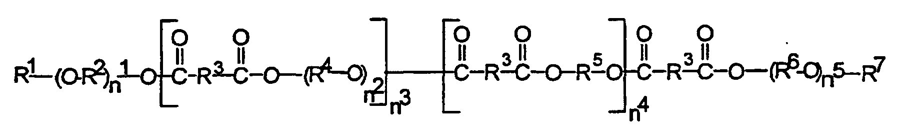 worin
- R¹ und R⁷: lineares oder verzweigtes C₁- bis C₁₈-Alkyl,
- R² und R⁶: Ethylen,
- R3: o-, m- oder p-Phenylen,
- R⁴: Ethylen,
- R⁵: Ethylen, 1,2-Propylen oder statistische Gemische von beliebiger Zusammensetzung von beiden,
- n¹ und n⁵: unabhängig voneinander eine Zahl zwischen 1 und 500,
- n2: eine Zahl von 10 bis 140,
- n³: eine Zahl von 0 bis 12,
- n⁴: eine Zahl von 7 bis 40,
bedeuten.

Bevorzugt bedeuten unabhängig voneinander
- R¹ und R⁷: lineares oder verzweigtes C₁- bis C₄-Alkyl,
- R³: p-Phenylen,
- n¹ und n⁵: eine Zahl von 3 bis 45,
- n²: eine Zahl von 18 bis 70,
- n³: eine Zahl von 0 bis 5,
- n4: eine Zahl von 8 bis 12,
- n³+n⁴: eine Zahl von 12 bis 18 oder von 25 bis 35.
Die Synthese der erfindungsgemäßen Oligoester erfolgt aus der Dicarbonsäure HOOC-R³-COOH bzw. deren Dimethylester, vorzugsweise Dimethylterephthalat, Ethylen- und/oder Propylenglykol, Polyethylenglykol und C₁- bis C₁₈-Alkylpolyethylenglykol, indem unter Zusatz eines Katalysators durch Erhitzen auf Temperaturen von 160 bis ca. 220 °C zunächst bei Normaldruck unter Abdestillieren des Methanols eine Umesterung durchgeführt und dann im Vakuum bei Temperaturen von 160 bis ca. 240 °C unter Abdestillieren überschüssiger Glykole kondensiert wird. Es eignen sich die Umesterungs- und Kondensationskatalysatoren des Standes der Technik, wie beispielsweise Titantetraisopropylat, Dibutylzinnoxid oder Antimontrioxid/Calciumacetat.

Diese Oligoester können in jede Art von Haarbehandlungsmittel eingearbeitet werden, beispielsweise in Shampoos, Creme-Rinses, Haarspülungen, Haarlotions, Haarwellmittel oder Aerosolschäume. Der Gehalt an Oligoester in diesen Haarpflegemitteln kann in breiten Grenzen schwanken und beträgt im allgemeinen 0,5 bis 5, vorzugsweise 0,5 bis 3, insbesondere 1 bis 2 Gew.-%, bezogen auf das Haarbehandlungsmittel.

Darüberhinaus enthalten die erfindunsgemäßen Haarbehandlungsmittel die hier üblichen Bestandteile, im wesentlichen anionische, nicht-ionische, kationische, zwitterionische oder amphotäre Tenside sowie Hilfs- und Zusatzstoffe wie Emulgatoren, Überfettungsmittel, biogene Wirkstoffe, Filmbildner, Konservierungsmittel, Perlglanzmittel, Farbstoffe.

Anionische Tenside sind insbesondere die folgenden Verbindungen sowie deren Mischungen:
Alkalisalze, Ammoniumsalze, Aminsalze und Salze von Aminoalkoholen folgender Verbindungen: Alkylsulfate, Alkylethersulfate, Alkylamid-sulfate und -äthersulfate, Alkylarylpolyethersulfate, Monoglyceridsulfate, Alkylsulfonate, Alkylamidsulfonate, Alkylarylsulfonate, α-Olefinsulfonate, Alkylsulfosuccinate, Alkyläthersulfosuccinate, Alkylamidsulfosuccinamate, Alkylsulfoacetate, Alkylpolyglycerin-carboxylate, Alkylphosphate, Alkylätherphosphate, Alkylsarcosinate, Alkylpolypeptidate, Alkylamidopolypeptidate, Alkylisäthionate, Alkyltaurate.

Salze von gesättigten und ungesättigten Fettsäuren, wie Oleinsäure, Ricinoleinsäure, Palmitinsäure, Stearinsäure, Kopraölsäuresalze oder hydrierte Kopraölsäuresalze sowie Alkylpolyethoxycarboxylate.

Kationische Tenside sind insbesondere quartäre Ammoniumverbindungen mit langer Kette, Alkylpyridiniumsalze, Fettamine von Polyäthern, Imidazolinderivate.

Nichtionische Tenside sind insbesondere polyäthoxylierte, polypropoxylierte oder polyglycerinierte Ether von Fettalkoholen, polyäthoxilierte, polypropoxylierte und polyglycerinierte Fettsäureester, polyäthoxylierte Ester von Fettsäuren und von Sorbit, polyäthoxilierte oder polyglycerinierte Fettamide.

Amphotere Tenside sind insbesondere Alkylamino-mono- und dipropionate, Betaine, wie N-Alkylbetaine, N-Alkylsulfobetaine, N-Alkylamidobetaine, Cycloimidiniumverbindungen, wie Alkylimidazoline, Asparaginderivate, wobei die Alkylgruppe in diesen oberflächenaktiven Mitteln vorzugsweise 1 bis 22 Kohlenstoffatome aufweist.

Die erfindungsgemäßen Haarbehandlungsmittel können als Hilfs- und Zusatzstoffe Emulgatoren wie etwa nicht-ionische Emulgiermittel, beispielsweise oxyäthylierte oder polyglycerinierte Fettalkohole, z.B. Oleinalkohol, polyoxyethylierte mit 10 bis 30 Mol Äthylenoxid, Stearylalkohol mit 10 bis 15 oder 20 Mol Ethylenoxid, Oleinalkohol, polyglyceriniert mit 4 Mol Glycerin, synthetische Fettalkohole mit 9 bis 15 C-Atomen, polyoxyäthyliert mit 5 bis 10 Mol Äthylenoxid, Sorbitanester, Monoglyceride, Polysorbate, Polyethylenglycolmono/difettsäureester, hochethoxilierte Fettsäureester sowie hochmolekulare Siliconverbindungen, wie z.B. Dimethylpolysiloxan und Phosphorsäureester in einer Menge von 1 bis 25 Gew.-% enthalten oder ionische Emulgiermittel, wie gegebenenfalls oxyethylierte Alkylsulfate, z.B. Natriumlaurylsulfat, Ammoniumlaurylsulfat, Ammoniumlaurylsulfat, Natriumcetylstearylsulfat, Triethanolaminstearylsulfat, Monoäthanolaminlaurylsulfat, Natriumlaurylethersulfat, und Monoethanolaminlaurylethersulfat, wobei diese letzteren Emulgiermittel in Konzentrationen zwischen 0.5 und 15 Gew.-% vorliegen. Als Überfettungsmittel können Substanzen wie beispielsweise polyethoxilierte Lanolinderivate, Lecithinderivate und Fettsäurealkanolamide verwendet werden, wobei die letzeren gleichzeitig als Schaumstabilisatoren dienen. Als Verdickungsmittel werden bevorzugt gehärtetes Rizinusöl, Salze von langkettigen Fettsäuren, vorzugsweise in Mengen bis zu 5 Gew.-% und insbesondere in Mengen von 0,5 bis 2 Gew.-%, eingesetzt, beispielsweise Natrium-, Kalium-, Aluminium-, Magnesium- und Titan-Stearate oder die Natrium und/oder Kalium-Salze der Behensäure, sowie Polysaccharide, insbesondere Xanthan-Gum, Guar-Guar, Agar-Agar, Alginate und Tylosen, Carboxymethylcellulose und Hydroxyethylcellulose, ferner höhermolekulare Polyethylenglycolmono- und diester von Fettsäuren, Polyacrylate, Polyvinylalkohol und Polyvinylpyrrolidon sowie Elektrolyte wie Kochsalz und Ammoniumchlorid.

Unter biogenen Wirkstoffen sind beispielsweise Pflanzenextrakte, Eiweishydrolysate und Vitaminkomplexe zu verstehen. Gebräuchliche Filmbildner sind beispielsweise Chitosan, mikrokristallines Chitosan, quarterniertes Chitosan, Polyvinylpyrrolidon, Vinylporrolidon-Vinylacetat-Copolymerisate, Polymere der Acrylsäurereihe, quarternäre Cellulose-Derivate und ähnliche Verbindungen. Als Konservierungsmittel eignen sich beispielsweise Phenoxyethanol, Formaldehydlösung, Parabene, Pentadiol oder Sorbinsäure. Als Perglanzmittel kommen beispielsweise Glycoldistearinester wie Ethylenglycoldistearat, aber auch Fettsäuremonoglycolester oder Triethylenglykoldistearat in Betracht. Als Farbstoffe können die für kosmetische Zwecke geeigneten und zugelassenen Substanzen verwendet werden, wie sie beispielsweise in der Publikation "Kosmetische Färbemittel" der Farbkommisision der Deutschen Forschungsgemeinschaft, veröffentlicht im Verlag Chemie, Weinheim, 1984, S. 81 - 106 zusammengestellt sind. Diese Farbstoffe werden üblicherweise in Konzentrationen von 0,001 bis 0,1 Gew.-%, bezogen auf die gesamte Mischung, eingesetzt. Die gewünschte Viskosität kann durch Zugabe von Wasser und/oder organischen Lösungsmitteln oder durch Zugabe einer Kombination aus organischen Lösungsmitteln und Verdickungsmitteln eingestellt werden.

Mit dem erfindungsgemäßen Einsatz der Oligoester in Haarbehandlungsmitteln, insbesondere in Haarschampoos, Haarspülungen und Haarkuren, lassen sich Schädigungen der Haare beseitigen, die Rückfettung der Haare verzögern und die Haaravivage, sowie der Haarglanz verbessern. Die nachfolgenden Beispiele sollen den Gegenstand der Erfindung näher erläutern, ohne ihn darauf einzuschränken.

### Beispiele

| Haarshampoo I | | |
|---|---|---|
| A | Genapol LRO flüssig | 35.00 % |
| B | Genapol AMG | 8.00 % |
| | SRC-Polymer | 1.00. % |
| | Parfümöl | 0.30 % |
| | Gelita Sol C | 1.00 % |
| | Wasser | 42.20 % |
| | Farbstofflösung | q.s |
| | Konservierungsmittel | q.s. |
| | Genagen CAB | 12.00 % |
| | Genapol L-3 | 1.00 % |
| C | Natriumchlorid | |

### Herstellung:

Nacheinander die Komponenten B in A einrühren,
Gegebenfalls den pH-Wert regulieren
Abschließend die Viskosität mit C einstellen.

| Haarshampoo II | | |
|---|---|---|
| A | Genapol LRO flüssig | 40.00 % |
| | SRC-Polymer | 1.50 % |
| B | Parfümöl | 0.30 |
| C | Wasser | 51.00 % |
| D | Natriumhydroxid | 0.20 % |
| E | Hostapon LEC | 4.20 % |
| | Genapol L-3 | 2.00 % |
| | Farbstofflösung | q.s |
| | Konservierungsmittel | q.s. |
| F | Natriumchlorid | 1.00 % |

### Herstellung:

B mit A mischen
D in C lösen, dann in 1 einrühren
Nacheinander die Komponenten von E in 1 einrühren
Gegebenenfalls den pH-Wert regulieren
Abschließend die Viskosität mit F einstellen

| Haarshampoo III | | |
|---|---|---|
| A | Genapol LRO flüssig | 35.00 % |
| | SRC-Polymer | 1.45 % |
| | Hostapon LEC | 5.30 % |
| B | Natronlauge (50 %ig) | 0.70 % |
| | Parfümöl | 0.30 % |
| | Genapol L-3 | 1.00 % |
| | Wasser | 47.00 % |
| | D-Panthenol | 0.50 % |
| | Genagen CAB | 8.00 % |
| | Farbstofflösung | q.s. |
| | Konservierungsmittel | q.s. |
| C | Natriumchlorid | |

### Herstellung:

Nacheinander die Komponenten von A in B einrühren
Gegebenenfalls den pH-Wert regulieren
Abschließend die Viskosität mit C einstellen

| Haarshampoo IV | | |
|---|---|---|
| A | Genapol LRO flüssig | 30.00 % |
| | SRC-Polymer | 1.00 % |
| B | Genapol SBE | 6.00 % |
| | Parfümöl | 0.30 % |
| | Wasser | 54.00 % |
| | Genapol L-3 | 2.00 % |
| | Genagen CAB | 5.00 % |
| | Farbstofflösung | q.s. |
| | Konservierungsmittel | q.s. |
| C | Natriumchlorid | 1.70 % |

### Herstellung:

Die Komponenten von B nacheinander in A einrühren.
Gegebenenfalls den pH-Wert regulieren.
Abschließend die Viskosität mit C einstellen.

| Haarshampoo V | | |
|---|---|---|
| A | Genapol LRO flüssig | 30.00 % |
| | SRC-Polymer | 1.00 % |
| B | Genapol AMG | 8.00 % |
| | Hostapon KCG | 5.00 % |
| | Parfümöl | 0.30 % |
| | Wasser | 45.70 % |
| | Farbstofflösung | q.s. |
| | Konservierungsmittel | q.s. |
| | Genagen CAB | 6.00 % |
| | Genapol L-3 | 2.00 % |
| C | Natriumchlorid | 2.00 % |

### Herstellung:

Nacheinander die Komponenten von B in A einrühren.
Gegebenenfalls den pH-Wert regulieren
Abschließend die Viskosität mit C einstellen.

### Chemische Bezeichnung der eingesetzten Handelsprodukte

- ®Genapol LRO: Laurylalkoholethersulfat (Clariant GmbH)
- ®Genapol AMG: Magnesium Amidether Sulfat (Hoechst AG)
- ®Gelita Sol C: Hydrolysiertes Collagen (Dt. Gelatine Fab.)
- ®Genagen CAB: Cocamidopropyl Betain (Hoechst AG)
- ®Genapol L-3: Laurylalkohol mit 3 mol Ethylenoxid (Hoechst AG)
- ®Hostapon LEC: Lauroylethercarbonsäure (Hoechst AG)
- ®Genapol SBE: Laurylsulfobemsteinsäureester
- ®Hostapon KCG: Natrium Cocoyl Glutamate (Hoechst AG)
- SRC-Polymer:: nichtionischer Polyester (Soil Release Polyester)

## Patentansprüche

1. Haarbehandlungsmittel, enthaltend 0,5 bis 5 Gew.-% Oligoester der Formel 1 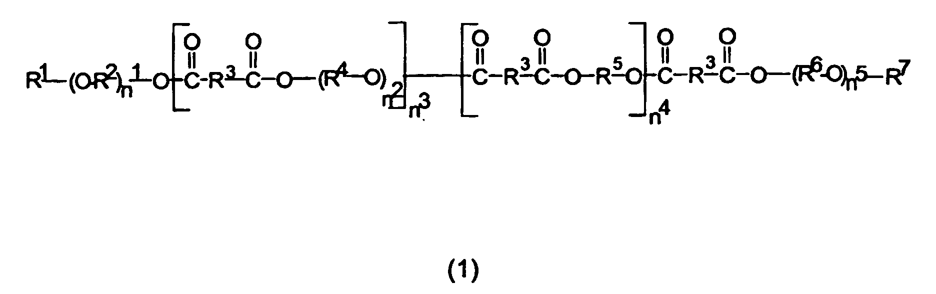 worin
R¹ und R⁷ lineares oder verzweigtes C₁- bis C₁₈-Alkyl,
R² und R⁶ Ethylen,
R³ o-, m- oder p-Phenylen,
R⁴ Ethylen,
R⁵ Ethylen, 1,2-Propylen oder statistische Gemische von beliebiger Zusammensetzung von beiden,
n¹ und n⁵ unabhängig voneinander eine Zahl zwischen 1 und 500,
n² eine Zahl von 10 bis 140,
n³ eine Zahl von 0 bis 12,
n⁴ eine Zahl von 7 bis 40,
bedeuten.

2. Haarbehandlungsmittel nach Anspruch 1, **dadurch gekennzeichnet, daß** das Haarbehandlungsmittel einen Oligoester der Formel 1 enthält, worin
R¹ und R⁷ lineares oder verzweigtes C₁- bis C₄-Alkyl,
R³ p-Phenylen,
n¹ und n⁵ eine Zahl von 3 bis 45,
n² eine Zahl von 18 bis 70,
n³ eine Zahl von 0 bis 5,
n⁴ eine Zahl von 8 bis 12,
n³+n⁴ eine Zahl von 12 bis 18 oder von 25 bis 35
bedeuten.

3. Haarbehandlungmittel nach Anspruch 1, enthaltend 1 bis 5 Gew.-% des Oligoesters, bezogen auf das Haarbehandlungsmittel.

## Claims

1. A hair-treatment composition comprising from 0.5 to 5% by weight of oligoesters of the formula 1 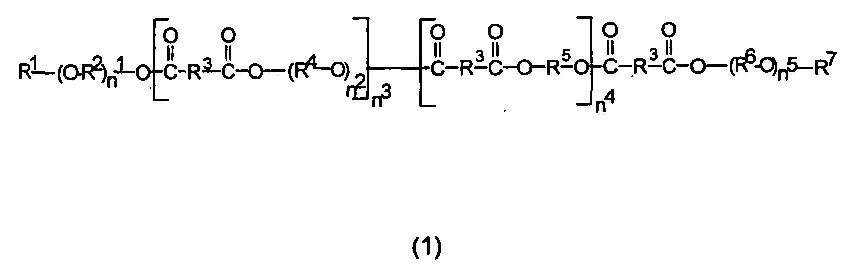 in which
R¹ and R⁷ are linear or branched C₁- to C₁₈-alkyl,
R² and R⁶ are ethylene,
R³ is o-, m- or p-phenylene,
R⁴ is ethylene,
R⁵ is ethylene, 1,2-propylene or random mixtures of any composition of the two,
n¹ and n⁵ independently of one another are a number between 1 and 500,
n² is a number from 10 to 140,
n³ is a number from 0 to 12,
n⁴ is a number from 7 to 40.

2. The hair-treatment composition as claimed in claim 1, wherein the hair-treatment composition comprises an oligoester of the formula 1 in which
R¹ and R⁷ are linear or branched C₁- to C₄-alkyl,
R³ is p-phenylene,
n¹ and n⁵ are a number from 3 to 45,
n² is a number from 18 to 70,
n³ is a number from 0 to 5,
n⁴ is a number from 8 to 12,
n³+n⁴ is a number from 12 to 18 or from 25 to 35.

3. The hair-treatment composition as claimed in claim 1, comprising from 1 to 5% by weight of the oligoester, based on the hair-treatment composition.

## Revendications

1. Produit pour le traitement des cheveux, contenant de 0,5 à 5 % en poids d'un oligoester de formule 1 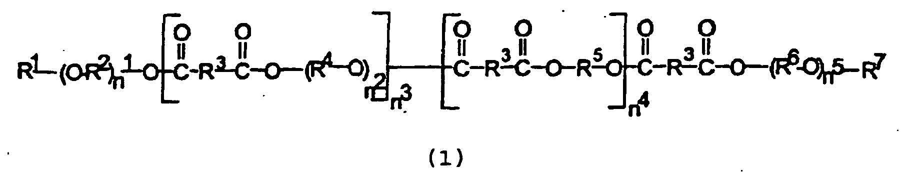 dans laquelle
R¹ et R⁷ sont des groupes alkyle en C₁ à C₁₈ à chaîne droite ou ramifiée,
R² et R⁶ sont des radicaux éthylène,
R³ est le radical o-, m- ou p-phénylène,
R⁴ est le radical éthylène,
R⁵ est le radical éthylène, 1,2-propylène, ou un mélange statistique de composition quelconque des deux,
n¹ et n⁵ représentent chacun indépendamment de l'autre un nombre compris entre 1 et 500,
n² est un nombre de 10 à 140,
n³ est un nombre de 0 à 12,
n⁴ est un nombre de 7 à 40.

2. Produit pour le traitement des cheveux selon la revendication 1, **caractérisé en ce que** le produit pour le traitement des cheveux contient un oligoester de formule 1 dans laquelle
R¹ et R⁷ sont des groupes alkyle en C₁ à C₄ à chaîne droite ou ramifiée,
R³ est le radical p-phénylène,
n¹ et n⁵ sont des nombres de 3 à 45,
n² est un nombre de 18 à 70,
n³ est un nombre de 0 à 5,
n⁴ est un nombre de 8 à 12,
n³+n⁴ est un nombre de 12 à 18 ou de 25 à 35.

3. Produit pour le traitement des cheveux selon la revendication 1, contenant 1 à 5 % en poids de l'oligoester par rapport au produit pour le traitement des cheveux.
